# EUROPEAN PATENT APPLICATION

(11) **EP 1 350 844 A2**
(43) Date of publication of application: **08.10.2003**
(21) Application number: 03251866.4
(22) Date of filing: 25.03.2003
(51) Int. Cl.: C12N 9/42, C12N 15/56

(54) **Xyloglucanase, polynucleotide encoding the enzyme, and method of preparing the enzyme**

(30) Priority: 25.03.2002 JP 2002083433
(71) Applicant: National Institute of Advanced Industrial Science and Technology, Tokyo 100-8921 (JP)
(72) Inventor: Yaoi, Katsuro c/o National Inst. of Adv. Indust., 1-chome,Tsukuba-shi,Ibaraki-ken 305-8566 (JP); Mitsuishi, Yasushi National Inst. of Adv. Indust., 1-chome,Tsukuba-shi,Ibaraki-ken 305-8566 (JP)
(74) Representative: Daniels, Jeffrey Nicholas

(57) **Abstract**

The present invention provides a novel xyloglucan oligosaccharide-degrading enzyme having a different degradation mechanism from known enzymes. This enzyme specifically cleaves the second β-glucoside linkage, counted from the reducing end among the β-glucoside linkage constituting the principal chain of xyloglucan oligosaccharide. In the present invention, the enzyme is collected from microorganisms belonging to *Geotrichum* species, and the amino acid and base sequences of the enzyme and polynucleotide encoding the enzyme are determined. Thus, high-purity polypeptides having the xyloglucan oligosaccharide-degradation activity can be prepared at low cost through a genetic engineering process.

## Description

### FIELD OF THE INVENTION

The present invention relates to a novel xyloglucan oligosaccharide-degrading enzyme, a polypeptide comprising a specific amino acid sequence having a xyloglucan oligosaccharide-degradation activity, a polynucleotide encoding the polypeptide, a recombinant vector containing the polynucleotide, a transformant transformed by the recombinant vector, and a method of culturing the transformant to prepare the polypeptide.

### BACKGROUND OF THE INVENTION

Xyloglucan is a heteropolysaccharide comprising monosaccharides such as glucose, xylose, galactose, fucose and arabinose. The name "xyloglucan" is derived from its basic structure in which xylose branches formed by α-1,6 xyloside linkages are frequently linked to the principal chain having a number of glucoses linked thereto by β-1,4-glucoside linkages. Xyloglucan plays an important role as not only a component of the primary cell walls of a plant but also as a polysaccharide stored in seeds of tropical leguminous plants represented by *Tamarindus indica*, and its structure and function are regarded as noteworthy research subjects. In particular, it is believed that xyloglucan in the primary cell walls of a plant is closely related to cellulose, and its morphological status relative to cellulose is a critical factor in the elongation and/or morphological differentiation of plant cells. Among conventional techniques for use in analyzing the roles of xyloglucan, various types of glycosidases capable of degrading xyloglucan serve as an important and powerful analyzing tool.

The known glycosidases capable of degrading xyloglucan, which comprises monosaccharides such as glucose, xylose, galactose, fucose and arabinose and has a principal chain structure similar to cellulose, as described above, includes endo-1,4-β-D-glucanase, β-galactosidase, isoprimeverose-producing-oligoxyloglucan-degrading enzyme, α-xylosidase, β-glucosidase and α-fucosidase. These enzymes, originated from various sources, have been used to analyze the structure and function of xyloglucan.

However, as assumable from the complicated structure of xyloglucan, the known glycosidases do not cleave the linkages to completion, and a number of uncleaved linkages are left. Thus, the need for developing a novel glycosidase having a different degradation mechanism from the known enzymes still exists.

### SUMMARY OF THE INVENTION

In view of the fact that xyloglucan includes predominant glucoside linkages which cannot be cleaved directly by the known glycosidases and that enzymes generally have a high substrate specificity, the inventors carried out the screening of natural microorganisms capable of utilizing xyloglucan as a carbon source. Xyloglucan oligosaccharide substrates having various structures were used to screen the microorganisms for xyloglucan-degrading enzyme systems capable of degrading xyloglucan through unknown degradation mechanisms. As a result, the inventors found that one strain belonging to *Geotrichum* species *(Geotrichum-species* strain M128), a yeast fungi and a strain known to produced high-purity xyloglucan nona-oligosaccharide in large quantities, produces a novel xyloglucan oligosaccharide-degrading enzyme. Based on this knowledge, the inventors analyzed the amino acid sequence of the enzyme and the polynucleotide encoding the enzyme, thereby accomplishing the present invention.

Specifically, according to a first aspect of the present invention, there is provided an enzyme which specifically cleaves the second β-glucoside linkage, counted from the reducing end among the β-glucoside linkages constituting the principal chain of xyloglucan oligosaccharide.

According to a second aspect of the present invention, there is provided a polypeptide comprising an amino acid sequence shown in SEQ ID NO: 14, or an amino acid sequence having one or more amino acid deletions, additions, insertions or substitutions relative to the amino acid sequence shown in SEQ ID NO: 14. The polypeptide has a xyloglucan oligosaccharide-degradation activity.

The polypeptide set forth in the second aspect of the present invention may further include methionine at the N-terminus thereof.

The polypeptide set forth in the second aspect of the present invention may further include a signal sequence.

According to a third aspect of the present invention, there is provided a polypeptide comprising an amino acid sequence shown in SEQ ID NO: 12, or an amino acid sequence having one or more amino acid deletions, additions, insertions or substitutions relative to the amino acid sequence shown in SEQ ID NO: 12. This polypeptide serves as a precursor of a polypeptide having a xyloglucan oligosaccharide-degradation activity.

The amino acid sequence of the above polypeptide including methionine at its N-terminus in the second aspect of the present invention may be an amino acid sequence shown in SEQ ID NO: 18.

According to a fourth aspect of the present invention, there is provided a polynucleotide encoding the polypeptide set forth in either the second or third aspects of the present invention.

According to a fifth aspect of the present invention, there is provided a polynucleotide comprising a base sequence shown in SEQ ID NO: 13, or a base sequence having one or more nucleic acid deletions, additions, insertions or substitutions relative to the base sequence shown in SEQ ID NO: 13. This polynucleotide expresses a polypeptide having a xyloglucan oligosaccharide-degradation activity.

The polynucleotide set forth in the fifth aspect of the present invention may further include an initiation codon.

The polynucleotide set forth in the fifth aspect of the present invention may further include a base sequence corresponding to a signal peptide sequence.

According to a sixth aspect of the present invention, there is provided a polynucleotide comprising a base sequence shown in SEQ ID NO: 11, or a base sequence having one or more nucleic acid deletions, additions, insertions or substitutions relative to the base sequence shown in SEQ ID NO: 11. This polynucleotide expresses a precursor of a polypeptide having a xyloglucan oligosaccharide-degradation activity.

According to a seventh aspect of the present invention, there is provided a polynucleotide which hybridizes under stringent conditions to the polynucleotide set forth in the fifth or sixth aspects of the present invention.

According to an eighth aspect of the present invention, there is provided a polynucleotide having homology to the polynucleotide set forth in any one of the fifth to seventh aspects of the present invention.

According to a ninth aspect of the present invention, there is provided a polynucleotide which is a degenerate of the polynucleotide set forth in any one of the fifth to seventh aspects of the present invention.

According to a tenth aspect of the present invention, there is provided a recombinant vector containing the polynucleotide set forth in any one of the fourth to ninth aspects of the present invention.

According to an eleventh aspect of the present invention, there is provided a transformant containing the recombinant vector set forth in the tenth aspect of the present invention.

According to a twelfth aspect of the present invention, there is provided a method of preparing a polypeptide, wherein the transformant set forth in the eleventh aspect of the present invention is cultured, and then a polypeptide corresponding to a resultingly recombined polynucleotide is collected from the cultured transformant.

In the method set forth in the twelfth aspect of the present invention, the polypeptide may have a xyloglucan oligosaccharide-degradation activity. Further, the xyloglucan oligosaccharide-degradation activity is operative to specifically cleave the second β-glucoside linkage, counted from the reducing end among the β-glucoside linkages constituting the principal chain of xyloglucan oligosaccharide.

The terms "comprising (comprise)," "including (include)" or "having (have)," for example in "polypeptide comprising an amino acid sequence" or "polynucleotide comprising a base sequence" are used herein to encompass not only a polypeptide or polynucleotide consisting of an amino acid or base sequence designated herein, but also any other polypeptide or polynucleotide into which such a designated sequence is incorporated as a part thereof.

The xyloglucan oligosaccharide-degrading enzyme of the present invention has a function of specifically cleaving the second β-glucoside linkage, counted from the reducing end among the β-glucoside linkages constituting the principal chain of xyloglucan oligosaccharide.

This enzyme is preparing from a *Geotrichum-species* strain M128, which is deposited as *Geotrichum* sp. strain M128, FERM P-16454 in the International Patent Organism Depositary, National Institute of Advanced Industrial Science and Technology, Japan (1 October 1997).

Typically, in a process of preparing the enzyme of the present invention from the *Geotrichum-species* strain M128, the strain is cultured aerobically at 20 to 30°C for 4 to 10 days by use of a liquid or solid medium which comprises a carbon source consisting of tamarind seed xyloglucan, and a nitrogen source including an inorganic or organic nitrogen source, such as nitrate salt, ammonium salt, peptone or yeast extract, and a small amount of metallic salts. The enzyme of the present invention is an extracellular enzyme excreted from the cells of the strains producing it. Thus, after culturing, a top supernatant obtained from the liquid medium through filtration or centrifugation, or a solution extracted from the solid medium by use of water or suitable inorganic salt, may be used as a crude enzyme solution. This crude enzyme solution contains the enzyme of the present invention and various different types of enzymes having a xyloglucan-degrading activity, and these different types of enzymes should be removed therefrom. These co-existing glycosidase activities can be eliminated through conventional chromatography.

The enzyme (purified product) of the present invention obtained through the above process has the following properties.

(1) Molecular Mass and Isoelectric Point: The purified enzyme of the present invention has a molecular mass of about 96 KDa and an isoelectric point pH of about 6.0.

(2) Action: The purified enzyme of the present invention acts on xyloglucan oligosaccharides having a polymerization degree of 3 or more in the principal chain among xyloglucan-originated oligosaccharides produced by partially degrading xyloglucans of various origins with endo-β-1,4-glucanase, to specifically cleave the second β-glucoside linkage counted from the reducing end among the β-glucoside linkages constituting the principal chain of the oligosaccharide.

If any hydroxyl group other than a 4-hydroxyl group participating in the linkage of the glucose residue at the reducing end of the principal chain is modified, the enzyme of the present invention cannot cleave the target β-glucoside linkage. Further, in the xyloglucan oligosaccharide, if among hydroxyl groups of the xylose side chain a 2-hydroxyl group linked to the third glucose residue counted from the reducing end glucose of the principal chain is modified, the enzyme of the present invention cannot cleave the second β-glucoside linkage counted from the reducing end.

With respect to a cellooligosaccharide having a polymerization degree of 4 or more and no xylose side chain, while the enzyme of the present invention cleaves the second β-glucoside linkage counted from the reducing end to produce cellobiose, the degradation speed or rate is significantly lower than that in a xyloglucan oligosaccharide, and the ratio therebetween is 1 : 200 or more.

While 1,4-β-D-glucan-cellobiohydrase (EC 3. 2. 1. 91) is known as an enzyme which degrades cellooligosaccharide having a polymerization degree of 4 or more to produce cellobiose, it cannot cleave any β-glucoside linkage constituting the principal chain of xyloglucan oligosaccharide having xylose side chains. As above, no enzyme specifically acting on the second β-glucoside linkage, counted from the reducing end among the β-glucoside linkages constituting the principle chain of xyloglucan oligosaccharide, is known. Thus, the enzyme of the present invention is a novel xyloglucan oligosaccharide-degrading enzyme having a new degradation mechanism.

(3) Acting pH and Optimum pH: When the enzyme of the present invention was applied to xyloglucan hepta-oligosaccharide used as a degradation substrate, at 45°C for 10 minutes, the observed acting pH and optimum pH of the enzyme were in the range of 3 - 5 and about 4.0, respectively.

(4) Stable pH Range: The stable pH of the enzyme of the present invention was in the range of 3.5 - 8.0 on the basis of the remaining activity of the enzyme after it was left at 45°C for 3 hours in a citrate-phosphate buffer solution.

(5) Action Temperature Range and Optimum Action temperature: When the enzyme of the present invention was applied to xyloglucan hepta-oligosaccharide used as a degradation substrate, at pH 4.0 for 10 minutes, the action of the enzyme was observed at a temperature of about 55°C or less, and the optimum action temperature was 50°C.

(6) Heat Stability: After the enzyme of the present invention was subjected to a heat treatment under various temperatures for 10 minutes in 50 mM acetate buffer solution (pH 4.0), the activity of the enzyme was fully maintained when the temperature of the heat treatment was 50°C or less, and about 10% of the activity was lost when the temperature was 55°C. Then, about 85% of the activity was lost when the temperature was 60°C.

(7) Inhibitor: Among various kinds of metal ions, a mercury ion at a concentration of 1 mM or more strongly inhibited the activity of the enzyme of the present invention.

(8) Purification method: The enzyme of the present invention could be purified to have SDS electrophoretic homogeneity by ultrafiltrating a cultivated top supernatant to concentrate and desalinate it, repeatedly subjecting the resulting concentrate to ion-exchange and adsorption chromatography using an anion exchanger PBE 94 column to further concentrate the fractions of the concentrate maintaining the activity, and subjecting the fractions to gel filtration using a TOYOPEAL HW55F column (made by TOSOH Corporation).

(9) Activity Measurement Method: In view of the feature of specifically degrading xyloglucan oligosaccharide, the activity of the enzyme of the present invention was determined by adding an appropriate amount of the enzyme to 0.5 ml (pH 4.0) of 30 mM aqueous solution of xyloglucan hepta-oligosaccharide prepared from tamarind gum while adjusting the total amount the solution to be 1 ml, causing a reaction therebetween at 45°C for 10 minutes, and measuring the produced reducing-sugar through a Nelson-Somogyi method. The amount of the enzyme capable of reducing one micro-mol of glucose per minute under the above measurement conditions was defined as 1 unit.

The novel xyloglucan oligosaccharide-degrading enzyme is a secretory protein comprising an amino acid sequence shown in SEQ ID NO: 12. In this sequence, the residues 1 - 23 are a signal sequence which are absent from the mature protein. The amino acid sequence of the mature protein is shown in SEQ ID NO: 14.

Thus, polypeptide suitable as the enzyme of the present invention has at least the amino acid sequence of SEQ ID NO: 14. Any homologous polypeptide comprising an amino acid sequence having one or more amino acid deletions, additions, insertions, or substitutions relative to the amino acid sequence of SEQ ID NO: 14 is also encompassed within the scope of the present invention as long as it has a xyloglucan oligosaccharide-degradation activity.

The polypeptide of the present invention includes a polypeptide precursor comprising the above signal sequence shown in SEQ ID NO: 12, or a homologous polypeptide precursor comprising an amino acid sequence having one or more amino acid deletions, additions, insertions, or substitutions relative to the amino acid sequence shown in SEQ ID NO: 12 and capable of producing a polypeptide having a xyloglucan oligosaccharide-degradation activity through splicing.

The polypeptide of the present invention can also be prepared by means of gene recombination. In this case, the polypeptide may additionally include methionine originated from initiation codon, at the N-terminus of an amino acid sequence shown in SEQ ID NO: 14, or may additionally include an amino acid residue originated from an expression vector, or a histidine tag for purifying a polypeptide to be obtained, at the C-terminus of the amino acid sequence. For example, it was verified that a polypeptide comprising an amino acid sequence shown in SEQ ID NO: 18 had a xyloglucan oligosaccharide-degradation activity.

A polynucleotide encoding the polypeptide of the present invention having a xyloglucan oligosaccharide-degradation activity includes a cloned gene from microorganisms producing the target enzyme, and any gene having homology to the gene. In the homologous gene, the homology may be at least 60% or more, preferably 80% or more, and more preferably 95% or more.

Preferably, the polynucleotide encoding the polypeptide of the present invention having a xyloglucan oligosaccharide-degradation activity is one of the following polynucleotides (DNA or RNA).

(1) A polynucleotide which comprises a base sequence shown in SEQ ID NO: 13, or a homologous base sequence having one or more nucleic acid deletions, additions, insertions, or substitutions, and encoding a polypeptide having a xyloglucan oligosaccharide-degradation activity.

(2) The polynucleotide set forth in the section (1) further including an initiation codon.

(3) The polynucleotide set forth in section (1) which further includes a base sequence corresponding to a signal sequence, or a homologous base sequence having one or more nucleic acid deletions, additions, insertions, or substitutions relative to the above base sequence, and having the capability of expressing a mature protein through splicing. Preferably, this polynucleotide comprises a base sequence shown in SEQ ID NO: 11.

In addition to the above polynucleotides, a polynucleotide which hybridizes under stringent conditions to any one of the polynucleotides set forth in the above sections (1) to (3), a polynucleotide having homology to any one of the above polynucleotides, and a polynucleotide which is a degenerate of any one of the above polynucleotides, are encompassed within the scope of the present invention, as long as polypeptides encoded by these polynucleotides have a xyloglucan oligosaccharide-degradation activity.

The term "stringent conditions" herein means, for example, keeping the polynucleotide at a temperature of 50°C to 60°C for 4 hours to one night in a 6 x SSC hybridization buffer (1 x SSC: 0.15 mol/L, 0.015 mol/L sodium citrate, pH 7.0) containing 0.5% SDS, 5 x Denhartd's (Denhartd's: 0.1% bovine serum albumin, 0.1% polyvinyl pyrrolidone, 0.1% Ficoll 400) and 100 µg/ml salmon sperm DNA.

The polynucleotide encoding the polypeptide of the present invention having a xyloglucan oligosaccharide-degradation activity can be obtained by cloning a gene of a microorganism producing the aforementioned xyloglucan oligosaccharide-degrading enzyme, for example, through the following method.

The novel xyloglucan oligosaccharide-degrading enzyme is first isolated from a microorganism producing the novel xyloglucan oligosaccharide-degrading enzyme and purified. Then, information about the partial amino acid sequence of the purified enzyme is obtained.

For example, the partial amino acid sequence may be determined by subjecting the purified enzyme protein directly to an amino acid sequence analyzer (e.g. Protein-sequencer 476A, made by Applied Biosystems) through an Edman degradation method (*Journal of Biological Chemistry,* 256, 7990-7997 (1981)), or may be effectively determined by subjecting the purified enzyme protein to limited hydrolysis while applying protein hydrolase thereto, isolating and purifying the obtained peptide fragments, and analyzing the amino acid sequence of the purified peptide fragments.

Then, a polynucleotide encoding a novel xyloglucan oligosaccharide-degrading enzyme is cloned in accordance with the obtained information about the partial amino acid sequence. Typically, the cloning can be carried out through a PCR method or a hybridization method.

For the PCR method or hybridization method, the method described in Sambrook and Russell, Molecular Cloning: A Laboratory Manual, Third edition, Cold Spring Harbor Laboratory Press (2001) may be used.

Particularly for the PCR method, the following method may be used. A cDNA of a microorganism producing the new xyloglucan oligosaccharide-degrading enzyme is first prepared as a template. Then, a PCR is performed using a synthesized oligonucleotide primer designed in accordance with the information about the partial amino acid sequence to obtain target polynucleotide fragments.

When the base sequence of the PCR-amplified DNA fragments is determined through a commonly used method such as a dideoxy-chain terminator method, a sequence corresponding to the partial amino acid sequence of the new xyloglucan oligosaccharide-degrading enzyme is found in the determined sequence in addition to the sequence of the synthesized oligonucleotide primer. That is, a part of the polynucleotide encoding the target enzyme can be obtained. Then, a full length polynucleotide encoding the new xyloglucan oligosaccharide-degrading enzyme over its entire length can be cloned through a hybridization method, or a 5'-RACE (Rapid Amplification of cDNA ends) and 3 '-RACE method using the obtained polynucleotide fragments as a probe.

In the present invention, the entire base sequence of the polynucleotide encoding the xyloglucan oligosaccharide-degrading enzyme was determined using the *Geotrichum* sp. strain M128 through a PCR method. This base sequence is shown in SEQ ID NO: 11.

The amino acid sequence encoded by this polynucleotide is shown in SEQ ID NO: 12. The 1st to 23rd amino acid residues counted from the N-terminus of this amino acid sequence indicates a signal sequence. The mature polypeptide has an amino acid sequence starting from the 24th lysine (SEQ ID NO: 14). The base sequence of the polynucleotide encoding this mature polypeptide is shown in SEQ ID NO: 13.

In addition to the base sequences shown in SEQ ID NOs: 11 and 13, additional base sequences corresponding to the amino acid sequences shown in SEQ ID NOs: 12 or 14 exist, due to the redundancy of the genetic code. These additional base sequences each encode the same amino acid sequence as the template sequence. Thus, for example, a degenerate of SEQ ID NO:11 would encode the same amino acid sequence of SEQ ID NO:11. Each of these additional base sequences are encompassed within the scope of the present invention, as well as degenerates of the homologous base sequences of the present invention defined above.

The target polynucleotide can also be obtained through chemical synthesis *(Gene,* 60(1), 115-127 (1987)) in accordance with the information about the amino acid sequence shown in SEQ ID NOs: 12 or 14 and the base sequence shown in SEQ ID NOs: 11 or 13.

All or a part of the novel xyloglucan oligosaccharide-degrading enzyme polynucleotide comprising the base sequence identified by using the *Geotrichum* sp. strain M128 may be used as a hybridization probe to select a polynucleotide having high homology to the new xyloglucan oligosaccharide-degrading enzyme polynucleotide comprising the base sequence shown in SEQ ID NO: 11, from a genomic DNA library or cDNA library of microorganisms producing different xyloglucan oligosaccharide-degrading enzymes.

The hybridization may be carried out under the aforementioned stringent conditions. For example, the genomic DNA library or cDNA library obtained from the microorganisms producing the new xyloglucan oligosaccharide is immobilized onto a nylon film, and the prepared nylon film is blocked at 65°C in a pre-hybridization solution containing a 6 x SSC, 0.5% SDS, 5 x Denhartd's, and 100 µg/ml salmon sperm DNA. Then, the hybridization solution containing ³²P labeled probes is added to the film, and kept at 65°C overnight. The nylon film is then rinsed in 6 x SSC at room temperature for 10 minutes, in 2 x SSC containing 0.1% SDS at room temperature for 10 minutes, and in 0.2 x SSC containing 0.1% SDS at 45°C for 30 minutes, and then subjected to autoradiography. As a result, a polynucleotide which specifically hybridizes to the probe can be obtained. Various types of homologous genes can also be obtained by changing the conditions of the rinse.

A PCR primer can be designed in accordance with the base sequence of the polynucleotide of the present invention. Through a PCR using the obtained primer, a polynucleotide fragment having high homology to the polynucleotide of the present invention can be obtained, and the full length polynucleotide can also be obtained.

In order to determine if the obtained polynucleotide encodes is the target polynucleotide encoding a polypeptide having a xyloglucan oligosaccharide-degradation activity, the presence of xyloglucan oligosaccharide- degradation activity in the obtained polynucleotide may be assumed from the difference or homology in their nucleic acid structures identified by comparing the determined base sequence with the base sequence or amino acid sequence of the new xyloglucan oligosaccharide-degrading enzyme of the present invention. Alternatively, the presence of xyloglucan oligosaccharide-degradation activity in the obtained polynucleotide may be directly measured from a polypeptide prepared from the obtained polynucleotide.

The following method is advantageous to prepare a polypeptide having a xyloglucan oligosaccharide-degradation activity by using the polynucleotide encoding the xyloglucan oligosaccharide-degrading enzyme of the present invention. An expression vector is first preparing comprising the polynucleotide of the present invention, and the obtained recombinant vector containing the polynucleotide is used to transform a host. Then, the obtained transformant is cultured under commonly used conditions to prepare the polypeptide having the new xyloglucan oligosaccharide-degradation activity. The mature polypeptide has no methionine residue at its N-terminus. Thus, the base sequence corresponding to the mature polypeptide may additionally incorporate an initiation codon (atg), and optionally a termination codon (taa/tag/tga) to provide a xyloglucan oligosaccharide-degradation activity in the mature polypeptide.

The host to be used in the above method may be a microorganism, animal cell or plant cell. The microorganism includes: bacteria such as *E coli, Bacillus* species, *Streptomyces* species, and Lactococcus species; yeasts such as *Saccharomyces* species, *Pichia* species, and *Kluyveromyces* species; and filamentous fungi such as *Aspergillus* species, *Penicillium* species, and *Trichoderma* species.

When an eucaryotic cell is used as the host, the mature polypeptide can be prepared through splicing, for example, even if a polynucleotide encoding a precursor polypeptide having the base sequence shown in SEQ ID NO: 11 corresponding to a signal sequence is used as the polynucleotide.

The expression and/or expressed products can be simply determined by use of an antibody to the new xyloglucan oligosaccharide-degrading enzyme. The expression can also be determined by measuring the enzyme activity.

The polypeptide having the new xyloglucan oligosaccharide-degradation activity may be purified from the transformant culture solution through an appropriate combination of centrifugation, UF concentration, salting-out, and various chromatographies such as an ion-exchange resin method, as described above. The polypeptide may also be advantageously purified, for example, by using a polynucleotide including a cay cay cay cay cay cay sequence (SEQ ID NO: 19) to encode the polypeptide to allow a histidine tag to be added to the C-terminus of the polypeptide to be produced, and optionally by using an expression vector having a histidine tag sequence and a termination codon to allow the above polynucleotide to be inserted in the upstream side of the 5'-terminus of the histidine tag.

The primary structure and polynucleotide structure of the xyloglucan oligosaccharide-degrading enzyme have been clarified by the present invention. Thus, the polynucleotide of the present invention can be used to introduce random mutations or site-specific mutations so as to obtain a polynucleotide comprising an amino acid sequence having at least one amino acid deletion, addition, insertion or substitution relative to the amino acid sequence of a natural xyloglucan oligosaccharide-degrading enzyme. This makes it possible to obtain polynucleotides encoding various xyloglucan oligosaccharide-degrading enzymes slightly different from each other in their optimum action temperature, stable action temperature, optimum pH, stable pH, or substrate-specific property while maintaining a desirable xyloglucan oligosaccharide-degradation enzyme activity, and to prepare various polypeptides having these enzyme activities through a genetic engineering process.

The random mutation may be introduced, for example, through a method of chemically treating DNA such as a method of inducing transition mutation or applying sodium bisulfite to substitute a cytosine base with a uracil base (*Proceedings of the National Academy of Sciences of the United States of America*, 79, 1408 - 1412 (1982)), a biological method such as a method of inducing base substitutions in the course of synthesizing double-stranded DNA under the presence of [α - S] dNTP (*Gene*, 64, 313 - 319 (1988)), or a method using PCR such as a method of adding manganese into a reaction system to perform a PCR so as to provide reduce accuracy in incorporating nucleotides (*Analytical Biochemistry*, 224, 347-353 (1995)).

The site-specific mutation may be introduced, for example, through a method utilizing amber mutation (gapped duplex method, *Nucleic Acids Research,* 12(24), 9441-9456 (1984)), a method utilizing a recognition site for a restriction enzyme (*Analytical Biochemistry*, 200, 81-88 (1992), *Gene,* 102, 67-70 (1991)), a method utilizing dut (dUTOase) and ung (uracil DNA glycosilase) mutation (Kunkel method, *Proceedings of the National Academy of Sciences of the United States of America*, 82, 488-492 (1985)), a method utilizing amber mutations using DNA polymerase and DNA ligase (Oligonucleotide-directed Dual Amber (ODA) method, *Gene,* 152, 271-275 (1995), Japanese Patent Laid-Open Publication No. 7-289262), a method utilizing a host inducing DNA repair system (Japanese Patent Laid-Open Publication No. 8-70874), a method utilizing a protein catalyzing a DNA strand exchange reaction (Japanese Patent Laid-Open Publication No. 8-140685), a PCR method using two kinds of mutagenic primers added with a recognition site for a restriction enzyme (USP 5,512,463), a PCR method using two kinds of primers and a double-stranded DNA vector having an inactivated drug-resistance gene (*Gene*, 103, 73-77 (1991)), and a PCR method utilizing amber mutations (International Patent Application No. WO 98/02535).

The site-specific mutation can be readily induced by using a commercially available kit. The commercially available kit includes Mutan®- G (made by Takara Shuzo Co., Ltd.) utilizing a gapped duplex method, Mutan® -K (made by Takara Shuzo Co., Ltd.) utilizing the a Kunkel method, Mutan®-Express Km (made by Takara Shuzo Co., Ltd.) utilizing an ODA method, and QuikChangeTM Site-Directed Mutagenesis Kit (made by STRATAGENE) using a mutagenic primer and DNA polymerase originated from *Pyrococcus furiosus*. The commercially available Kit utilizing PCR includes TaKaRa LA PCR in vitro Mutagenesis Kit (made by Takara Shuzo Co., Ltd.) and Mutan®-Super Express Km (made by Takara Shuzo Co., Ltd.).

As described above, the primary structure and polynucleotide structure of the xyloglucan oligosaccharide-degrading enzyme provided by the present invention allows high-purity polypeptides having a xyloglucan oligosaccharide-degradation activity to be prepared at a low cost through a genetic engineering process. The entire contents of the above publications are incorporated herein by reference. While the present invention will be described below in more detail with reference to Examples, the present invention is not limited thereto. The unit % herein means W/V % unless otherwise specified.

### DESCRIPTION OF THE PREFERRED EMBODIMENT

### EXAMPLE 1

20 ml of medium containing 1% of tamarind seed xyloglucan, 0.8% of peptone, 0.05% of magnesium sulfate, 0.2% of monopotassium phosphate, and 0.05% of yeast extract was put in an Erlenmeyer flask having a volume of 200 ml. The culture solution was sterilized in the usual manner, and then the *Geotrichum* sp. strain M128 (FERM P-16454) was inoculated into the solution. The solution was aerobically cultured at 30°C for 6 days, and then centrifuged and filtered. The activity of the obtained top supernatant was measured through the aforementioned activity-measurement method. The measured activity per 1 ml culture solution was 0.05 units.

### EXAMPLE 2

4000 ml of culture solution having the same composition as that in EXAMPLE 1 was prepared. The *Geotrichum* sp. strain M128 was inoculated into the culture solution. The culture solution was cultured in the same way as that in EXAMPLE 1, and the top supernatant of the resulting culture solution was obtained. A total enzyme activity in the culture solution was 216 units. The obtained top supernatant was concentrated and desalinated through ultrafiltration, and then purified through ion exchange/absorption chromatography using an anion exchanger column and gel filtration using a TOYOPEARL HW55F column. The purified enzyme had a SDS-electrophoretic homogeneity and an activity yield of 9.2% in the filtrated culture solution. The activity per 1 mg purified enzyme protein was 11.8 units.

### EXAMPLE 3

0.08 units of the purified enzyme obtained in EXAMPLE 2 was added to 1 ml of reaction solution containing 80 mg of xyloglucan hepta-oligosaccharide, and allowed to react at pH 4.0 and 45°C for 3 hours. After completion of the reaction, the resulting product was isolated and purified through Bio-Gel P-2 (made by Bio-Rad), and the structure of the purified product was analyzed. The product comprised two kinds of oligosaccharides: an oligosaccharide A having a polymerization degree of 3 and an oligosaccharide B having a polymerization degree of 4. The oligosaccharide A was degraded into equimolar isopremeverose and glucose by an isopremeverose-producing-oligoxyloglucan-degrading enzyme. The oligosaccharide B was degraded by the isopremeverose-producing-oligoxyloglucan-degrading enzyme to form only isopremeverose. This result proved that the enzyme cleaves the second β-glucoside linkage counted from the reducing end of the xyloglucan hepta-oligosaccharide among the β-glucoside linkages constituting the principal chain of the oligosaccharide serving as a substrate, to form branched tetra-saccharide and branched tri-saccharide.

### EXAMPLE 4

0.01 units of the purified enzyme obtained in EXAMPLE 2 was added to 5 mg each of various kinds of xyloglucan oligosaccharides shown in Table 1 in separate vessels, and each combination was allowed to react 18 hours. Then, the resulting oligosaccharides were analyzed through high-speed liquid chromatography. The result proved that the enzyme acts on xyloglucan oligosaccharides comprising a principal-chain having a polymerization degree of 3 or more, and specifically cleaves the second β-glucoside linkage counted from the reducing end among the β-glucoside linkages constituting the principal chain of the oligosaccharide. The result also proved that the enzyme cannot cleave the above second β-glucoside linkage if the glucose residue at the reducing end is modified, and that the side-chain xylose residue linked to the third glucose residue counted from the reducing end glucose of the principal chain has a key role, or the enzyme cannot cleave the above second β-glucoside linkage if the xylose is modified.

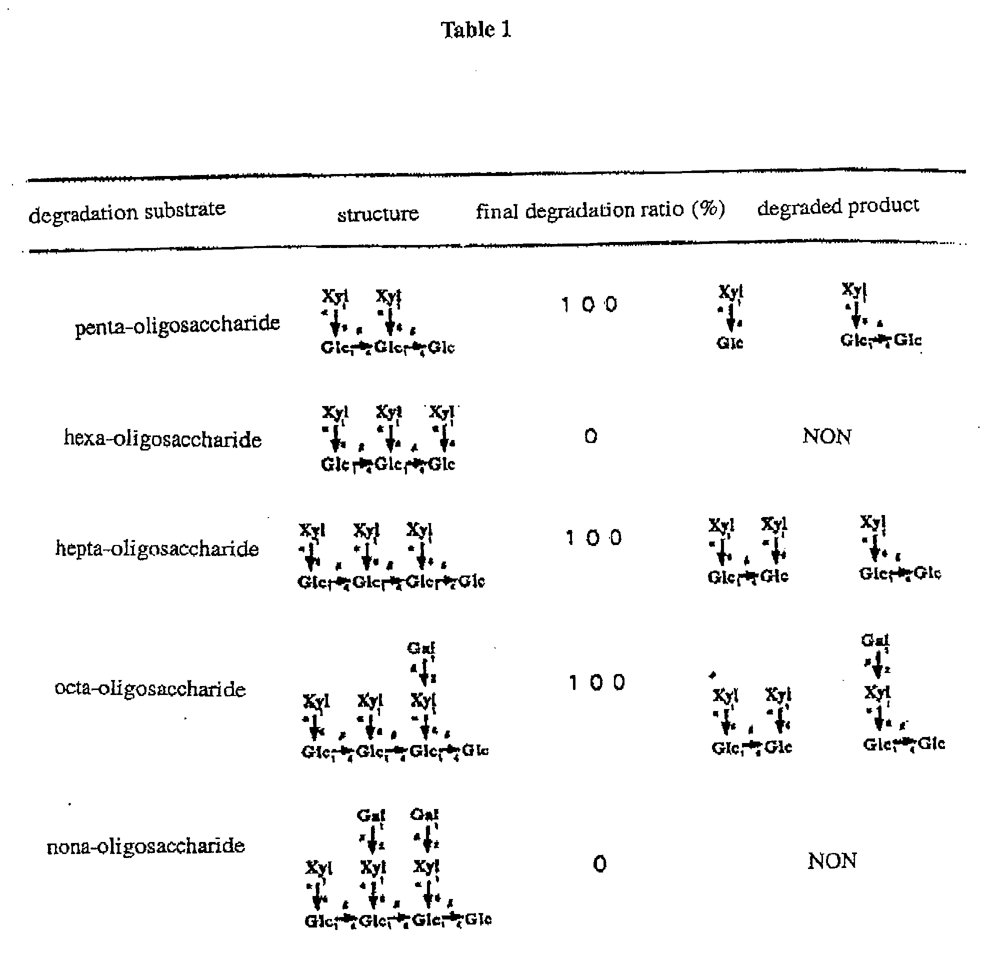

The isopremeverose-producing-oligoxyloglucan-degrading enzyme has been known as one of the enzymes that can directly cleave the 1,4-β-D-glucoside xyloglucan oligosaccharide constituting the principal chain of xyloglucan oligosaccharide having side chains. However, the enzyme degrades xyloglucan oligosaccharide into isopremeverose units in accordance with an exo-type degradation mechanism. The enzyme of the present invention has a completely different substrate-specific property and degradation mechanism from the above conventional enzyme in that it can directly cleave the second β-glucoside linkage counted from the reducing end among the β-glucoside linkages constituting the principal chain of xyloglucan oligosaccharide.

### EXAMPLE 5

Isolation of Polynucleotide Encoding Novel Xyloglucan Oligosaccharide-Degrading Enzyme Originated from *Geotrichum* sp. Strain M128.

The polynucleotide manipulation technique herein was in accordance with publications (e.g. Sambrook and Russell, Molecular Cloning: A Laboratory Manual, Third edition, Cold Spring Habor Laboratory Press, 2001) unless otherwise specified.

### 1. Determination of Partial Amino Acid Sequence

The purified sample of the xyloglucan oligosaccharide-degrading enzyme obtained in EXAMPLE 2 was subjected to Protein Sequencer (made by Applied Biosystems) to determine the 22-residue N-terminus amino acid sequence shown in SEQ ID NO: 1. Then, the purified sample of the xyloglucan oligosaccharide-degrading enzyme obtained in EXAMPLE 2 was degraded with lysyl-end peptidase (made by Wako Pure Chemical Industries, Ltd.). The obtained degradation product was subjected to reversed-phase liquid chromatography to obtain peptide fractions. Three of the separated peptide fractions were subjected to Protein Sequencer to determine the 9 to 12-residue internal amino acid sequences shown in SEQ ID NOs: 2, 3, and 4.

SEQ ID NO: 1:

Lys-Glu-His-Tyr-Glu-Phe-Lys-Asn-Val-Ala-Ile-Gly-Gly-Gly-G

ly-Tyr-Ile-Thr-Gly-Ile-Val-Ala

SEQ ID NO: 2:	Asp-Leu-Leu-Tyr-Ala-Arg-Thr-Asp-Ile-Gly-Gly-Ala

SEQ ID NO: 3:	Ala-Ser-Ala-Pro-Ser-Ala-Val-Phe-Ile-Trp-Gly-Thr

SEQ ID NO: 4: Val-Tyr-Gly-Arg-Val-Tyr-Leu-Gly-Thr

### 2. Preparation of Entire RNA

Culturing was conducted in the same way as that in EXAMPLE 1, and RNA was prepared in its entirety in the usual manner using an RNA isolation kit (FastRNA Kit-RED made by BIO 101) as directed by its attached operational instruction. Then, mRNA was purified using an mRNA preparation kit (QuickPrep mRNA Purification Kit made by Amersham Pharmacia Biotech). The obtained mRNA was used to synthesize cDNA with an oligo-dT-primer and reverse transcriptase by use of a cDNA synthesis kit (TimeSaver cDNA Synthesis kit made by Amersham Pharmacia Biotech Inc.).

### 3. Amplification through PCR

Based on the N-terminus-region amino acid sequence shown in SEQ ID NO: 1 and the internal amino acid sequence shown in SEQ ID NO: 3, oligonucleotide primers (a sense primer shown in SEQ ID NO: 5 and an antisense primer shown in SEQ ID NO: 6) were synthesized by a DNA synthesizer (made by Applied Biosystems Ltd.). The obtained oligonucleotides were used as PCR primers.

SEQ ID NO: 5 Sense Primer: 5'-GARCAYTAYGARTTYAARAAYGT-3'

SEQ ID NO: 6 Antisense Primer: 5'-GTNCCCCADATRAANACNGC-3'

These primers and the cDNA prepared from *Geotrichum* sp. strain M128 were used in a PCR performed on a GeneAmp PCR system 9700 (made by Applied Biosystems Ltd.).

The PCR solution contained 10 µl of 10 x PCR buffer solution (made by Takara Shuzo Co., Ltd.), 8 µ of dNTP mixture solution (2.5 mmol/l each, made by Takara Shuzo Co., Ltd.), 5 µl of 100 µmol/L sense primer, 5 µl of 100 µmol/L antisense primer, 71 µl of distilled water, 0.5 µl of cDNA solution (100 µg/ml), and 0.5 µl of EX-Taq DNA polymerase (made by Takara Shuzo Co., Ltd.).

The PCR conditions were as follows:
Stage 1: Denaturation (94°C for 5 minutes), 1 cycle
Stage 2: Denaturation (94°C for 1 minute), annealing (45°C for 1 minute), elongation (72°C for 2 minutes), 35 cycles
Stage 3: Elongation (72°C for 10 minutes), 1 cycle

The obtained DNA fragment of about 2 kbp was cloned into the pGEM-T Easy vector (made by Promega), and the base sequence of the cloned DNA was determined. Base sequences encoding the above partial amino acid sequence were found right after the sense primer and right before the antisense primer. A base sequence encoding the internal amino acid sequence shown in SEQ ID NO: 2 was also found.

### 4. Determination of 5'-Terminus and 3'-Terminus

Based on the above DNA base sequence, two different primers, shown in SEQ ID NOs: 7 and 8, were prepared as antisense primers for 5'-RACE (Rapid Amplification of cDNA ends). cDNA was used as a template to obtain a 5'-terminus DNA fragment through a 5'-RACE method, and the base sequence of the fragment was determined. Similarly, two different primers, shown in SEQ ID NOs: 9 and 10, were prepared as sense primers for 3'-RACE, and a 3'-terminus DNA fragment was obtained through the 3'-RACE method to determine the full length base sequence.

SEQ ID NO: 7: 5'-CGTACAGCAGGTCCTTGGTCTTTGG-3'

SEQ ID NO: 8: 5'-TAATGTACCCGCCGCCGCCGAT-3'

SEQ ID NO: 9: 5'-GGCAAGTTCTTCGTCTCGACCGAC-3'

SEQ ID NO: 10: 5'- CCAAGTCGGACGGCAAGAAGGCTA –3'

The determined base sequence of the cDNA encoding the xyloglucan oligosaccharide-degrading enzyme is shown in SEQ ID NO: 11. The obtained cDNA has a total length of 2646 base pairs and includes an open reading frame which extends from an initiation codon (atg) of 0th to 12th residues to a termination codon (taa) of 2556th to 2558th residues, thereby encoding a protein consisting of 812 amino acids, shown in SEQ ID NO: 12. The vicinity of the initiation codon fulfills a requirement of -3-g and +4-g, important for a translation initiation site. The N-terminus amino acid sequence (SEQ ID NO: 1) and the internal amino acid sequences (SEQ ID NOs: 2, 3 and 4) were found in this amino acid sequence.

The sequence (1st to 23rd residues) from the translation initiation site to the site just before the N-terminus amino acid of mature protein (SEQ ID NO: 1) is a signal sequence, which suggests that the enzyme of the present invention is a secretory protein.

The amino acid sequence of the mature protein is shown in SEQ ID NO: 14, and the base sequence of the polynucleotide encoding the mature protein is shown in SEQ ID NO: 13.

The present invention is not limited to polypeptides comprising the above sequences having a xyloglucan oligosaccharide-degradation activity and nucleotides encoding the polypeptides, but any other polypeptides having a longer length and comprising the polypeptide having a xyloglucan oligosaccharide-degradation activity and any nucleotides encoding such polypeptides are encompassed within the scope of the present invention.

### EXAMPLE 6

Construction of Expression Plasmid of New Xyloglucan Oligosaccharide-Degrading Enzyme in *E. coli.*

Based on the DNA base sequences encoding the N-terminus region amino acid sequence and C-terminus region amino acid sequence of the mature protein, a sense primer shown in SEQ ID NO: 15 and an antisense primer shown in SEQ ID NO: 16 were prepared. During this process, a restriction-enzyme-*Nde*I recognition sequence (catatg) was added to the 5' side of the sense primer in the N-terminus region, and a restriction-enzyme-*Bgl*II recognition sequence (agatct) was added to the 5' side of the antisense primer in C-terminus region. A plurality of bases were added to the 5' side of the restriction enzyme recognition sequence to provide an increased degradation ratio for the restriction enzyme.

SEQ ID NO: 15:

5'-TCGCTCGCGCATATGAAGGAGCACTACGAGTTCAAGAATG-3'

SEQ ID NO: 16: 5'-TGGAAGATCTCCTTTTTCACATAGCAGTGCGTGCCCTT-3'

These primers, and cDNA prepared from *Geotrichum* sp. strain M128 for use as a template, were used in a PCR performed using a GeneAmp PCR system 9700 (made by Applied Biosystems Ltd.) under the following conditions.

The PCR solution contained 10 µl of 10 x PCR buffer solution (made by Takara Shuzo Co., Ltd.), 8 µ of dNTP mixture solution (2.5 mmol/L each, manufactured by Takara Shuzo Co., Ltd.), 5 µl of 10 µmol/L sense primer, 5 µl of 10 µmol/L antisense primer, 71 µl of distilled water, 0.5 µl of cDNA solution (100 µg/ml), and 0.5 µl of EX-Taq DNA polymerase (made by Takara Shuzo Co., Ltd.).

The PCR conditions were as follows:
Stage 1: Denaturation (96°C for 1 minute), 1 cycle
Stage 2: Denaturation (96°C for 10 seconds), annealing and elongation (68°C for 4 minute), 30 cycles

The obtained DNA fragment of about 2.4 kbp was digested by restriction enzymes *Nde*I and *Bgl*II, and cloned into the pET29a(+) vector (manufactured by Novagen, Inc.) digested by restriction enzymes *Nde*I and *Bgl* II. After checking the adequacy of the base sequence, the cloned DNA was introduced into *E. coli* BL21-CodonPlus (DE3)-RP (made by STRATAGENE). The obtained transformant was shaking-cultured at 37°C in a LB medium containing 30 µg/ml kanamycin. Isopropyl-β-D-thiogalactopyranoside was added to the medium to induce production. The enzyme accumulated in the bacteria cells in the form of a protein inclusion body.

### EXAMPLE 7

Isolation, Solubilization and Unwinding of Protein Inclusion Body.

The cultivated cells were collected. Then, by using a protein extraction kit (BugBuster manufactured by Novagen), the collected cells were fractured, and the protein inclusion bodies were purified. The purified protein inclusion bodies were dissolved in 8M urea-1 mM dithiothreitol-50 mM Tris HCl - 1 mM ethylenediamine tetraacetic acid (pH 8.0) to adjust a protein concentration to about 1 mg/ml.

The solubilized top supernatant was dialyzed with 25 mM imidazole-HCl (pH 7.4) to remove urea and dithiothreitol so as to unwind polypeptide therein.

The amino acid sequence of the obtained polypeptide is shown in SEQ ID NO: 18, and the base sequence corresponding to this amino acid sequence is shown in SEQ ID NO: 17. The polypeptide comprises the amino acid of the mature polypeptide shown in SEQ ID NO: 14 having a C-terminus which additionally includes a histidine tag comprising 30 amino acid residues originated from the expression vector pET29a (+) and 6 histidine residues. The xyloglucan oligosaccharide-degradation activity of the polypeptide was examined by using the various xyloglucan oligosaccharides shown in Table 1 as a substrate. The results were completely the same as that of the enzyme obtained from the *Geotrichum* sp. strain M128. That is, the recombinant enzyme had an enzyme activity of specifically cleaving the second β-glucoside linkage counted from the reducing end among the β-glucoside linkages constituting the principal chain of the xyloglucan oligosaccharide.

From the above results, it was verified that the polynucleotide of the new xyloglucan oligosaccharide-degrading enzyme of the present invention can be used to prepare a recombinant enzyme using *E. coli.*

As mentioned above, the present invention provides a new enzyme which cleaves the second β-glucoside linkage, counted from the reducing end among the β-glucoside linkages constituting the principal chain of xyloglucan oligosaccharide. Further, the present invention discloses the amino acid sequence and the polynucleotide structure of the new enzyme. Thus, the present invention opens the way for achieving a new analysis technique of analyzing a structure and function of xyloglucan which must play an important role in elongation and morphological differentiation of plant cells.

SEQUENCE LISTING

<110> YAOI, Katsuro
      MITSUISHI, Yasushi

<120> NOVEL XYLOGLUCAN OLIGOSACCHARIDE-DEGRADING ENZYME, POLYNUCLEOTIDE
       ENCODING THE SAME, AND METHOD OF PREPARING THE ENZYME

<130> F166503

<150> JP 2002-83433
<151> 2002-03-25

<160> 19

<170> PatentIn version 3.2

<210> 1
<211> 22
<212> PRT
<213> Geotrichum sp. M128

<400> 1

Lys Glu His Tyr Glu Phe Lys Asn Val Ala Ile Gly Gly Gly Gly Tyr
1               5                   10                  15

Ile Thr Gly Ile Val Ala
            20

<210> 2
<211> 12
<212> PRT
<213> Geotrichum sp. M128

<400> 2

Asp Leu Leu Tyr Ala Arg Thr Asp Ile Gly Gly Ala
1               5                   10

<210> 3
<211> 12
<212> PRT
<213> Geotrichum sp. M128

<400> 3

Ala Ser Ala Pro Ser Ala Val Phe Ile Trp Gly Thr
1               5                   10

<210> 4
<211> 9
<212> PRT
<213> Geotrichum sp. M128

<400> 4

Val Tyr Gly Arg Val Tyr Leu Gly Thr
1               5

<210>  5
<211>  23
<212>  DNA
<213>  Artificial Sequence

<220>
<223>  Primer

<400>  5
garcaytayg arttyaaraa ygt                                              23

<210>  6
<211>  20
<212>  DNA
<213>  Artificial Sequence

<220>
<223>  Primer

<220>
<221>  misc_feature
<222>  (3)..(3)
<223>  n is a, c, g, or t

<220>
<221>  misc_feature
<222>  (15)..(15)
<223>  n is a, c, g, or t

<220>
<221>  misc_feature
<222>  (18)..(18)
<223>  n is a, c, g, or t

<400>  6
gtnccccada traanacngc                                                  20

<210>  7
<211>  25
<212>  DNA
<213>  Artificial Sequence

<220>
<223>  Primer

<400>  7
cgtacagcag gtccttggtc tttgg                                            25

<210>  8
<211>  22
<212>  DNA
<213>  Artificial Sequence

<220>
<223>  Primer

<400>  8
taatgtaccc gccgccgccg at                                                22

<210>  9
<211>  24
<212>  DNA
<213>  Artificial Sequence

<220>
<223>  Primer

<400>  9
ggcaagttct tcgtctcgac cgac                                              24

<210>  10
<211>  24
<212>  DNA
<213>  Artificial Sequence

<220>
<223>  Primer

<400>  10
ccaagtcgga cggcaagaag gcta                                              24

<210>  11
<211>  2646
<212>  DNA
<213>  Geotrichum sp. M128

<220>
<221>  CDS
<222>  (120)..(2558)

<400>  11
   aatcgccccg gtgttggctc atcgggccgc ggtgtcggtg ttgatacgag tacataattg      60
   ggactgtcgg tgcgattggc ccccaacttc accacacaac acctacacac tctttagat       119
   atg gtg gca gtc acc tcc ctc ggg aag gcg ctt act gcc ctt tcg att       167
   ctg gcg tcg ctc gcg gtc gcc aag gag cac tac gag ttc aag aat gtc       215
   gcg atc ggc ggc ggc ggg tac att acc ggg att gtc gcg cac cca aag       263
   acc aag gac ctg ctg tac gcg cgc acg gac att ggc ggc gcg tac cgc       311
   tgg gac gca ggc acg tcc aag tgg atc ccg ctc aac gac ttt atc gag 359
   gcg cag gac atg aac att atg ggc acc gag tcg atc gcg ctg gac ccc 407
   aac aac ccc gac agg ctg tac ctc gcg cag ggg cgc tat gtc ggc gac 455
   gag tgg gcg gcg ttc tat gtg tcc gaa gac cgc ggc cag tcg ttt aca 503
   atc tac gag tcg ccg ttc ccg atg ggc gcc aac gac atg gga cgc aac 551
   aat ggc gag cgc ctc gct gtc aac ccg ttc aac tcg aac gag gtc tgg 599
   atg ggt acg cgt aca gag ggt atc tgg aag agt tcg gac cgc gcc aag 647
   acc tgg aca aac gtc acg tcc atc ccg gac gcg ttc acc aac ggt atc 695
   gga tac acg tcg gtc att ttc gac ccc gaa cgt aat ggc acc atc tac 743
   gcg agc gcg act gcc ccg cag ggc atg tac gtc acg cac gac ggc ggt 791
   gtc tcg tgg gag cca gtg gcg ggc cag ccg tcc agc tgg ctc aac agg 839
   acc acg ggc gcg ttc ccg gac aag aag ccc gcg tcg atc gcg ccg cag 887
   ccc atg aaa gtc gct ctc acc ccc aac ttc ctc tac gtg act tac gcc 935
   gac tac cct ggt cca tgg ggc gtc acg ttc ggc gaa gtc tgg cgc cag 983
   aac cgc acc tcg ggc gcc tgg gac gac att act ccc cgc gtc ggc aac 1031
   tcg tcg cct gcc ccg tac aac aac cag acg ttc cct gcg ggc gga ttt 1079
   tgc ggt ctc agc gtc gac gcg acc aac ccc aac cgt ctc gtc gtc atc 1127
   acc ctc gac cgc gac ccc gga ccc gcc ctc gac agc atc tac ctc tca 1175
   acc gat gcc ggc gcg acc tgg aag gac gtc acc cag ctc tcg tcc ccg 1223
   tcc aac ctc gaa ggt aac tgg ggc cac ccg act aac gcg gcg cgg tac 1271
   aag gac ggc acg cct gtt ccg tgg ctc gac ttc aac aac ggt ccc cag 1319
   tgg ggg gga tac ggt gcg ccg cac ggt acg ccc ggc ctc acc aag ttt 1367
   ggc tgg tgg atg agc gct gtg ctt atc gat ccg ttc aac ccc gag cac 1415
   ctg atg tac ggc acg ggg gcg acc atc tgg gcg acc gac acg ctc tcc 1463
   cgt gtc gag aag gac tgg gcg ccg agc tgg tac ctc cag atc gac ggt 1511
   atc gag gag aat gcg atc ctg tcg ctc cgc tcg ccc aag agc ggc gcg 1559
   gcg ctc ctg tcg ggc atc ggt gac att agc ggc atg aag cac gac gac 1607
   ctc acc aag ccc cag aag atg ttt ggt gcg ccc cag ttc tcc aac ctc 1655
   gac agc atc gac gct gcg ggc aac ttc ccc aac gtt gtc gtc cgc gcc 1703
   gga tcc tcg gga cac gag tac gac agc gcg tgc gcg cgc ggt gcg tac 1751
   gcg act gac ggc gga gac gcg tgg acc atc ttc cct acc tgc cct cct 1799
   ggc atg aac gcg agc cac tac cag ggc agc acg att gca gtc gac gcg     1847
   agc ggc agc cag atc gtg tgg tcg acc aag ctt gac gag cag gcc tcg     1895
   gga ccg tgg tac tcg cac gac tat ggc aag acg tgg tct gtt ccc gct     1943
   ggc gac ctg aag gcc cag act gcc aat gtg ctc tcg gac aag gtc cag     1991
   gat ggc acg ttc tac gct acc gat ggc ggc aag ttc ttc gtc tcg acc     2039
   gac ggc ggg aag tcg tat gcc gcc aag ggc gcc gga ctt gtc act ggc     2087
   aca tcg ctc atg cct gcc gtg aac ccc tgg gtg gcc ggc gac gtc tgg     2135
   gtg cct gtt ccc gag ggc ggt ctc ttc cac tcg acc gac ttt ggc gcc     2183
   tcg ttc acg agg gta ggt acc gcc aac gcg acc ctc gtg agc gtc ggc     2231
   gcc ccc aag tcc aag tcg gac ggc aag aag gct agc gcg ccc tcc gcg     2279
   gtc ttc atc tgg ggc acc gac aag cct gga agc gac atc ggc ctg tac     2327
   cgc tcc gac gac aac ggc agc acc tgg acg cgc gtc aat gac cag gag     2375
   cac aac tac tcg ggc ccc acc atg atc gag gcc gac ccc aag gtc tac     2423
   ggg cgc gtg tat cta ggc acg aac ggc cgc ggt atc gtg tac gcc gac     2471
   ctt acc aac aag aag agc aac gag gag aag tcg acc gca aag tgc gcc     2519
   aac ggc cag aag ggc acg cac tgc tat gtg aaa aag taa atgggaagga      2568
   aagggcgaaa gggacatacc gcctggttat ttaaatgcat ctacatgtta cttaaaaaaa   2628
   aaaaaaaaaa aaaaaaaa                                                  2646

<210>  12
<211>  812
<212>  PRT
<213>  Geotrichum sp. M128

<400>  12

<210>  13
<211>  2367
<212>  DNA
<213>  Geotrichum sp. M128

<220>
<221>  mat_peptide
<222>  (1)..(2367)

<220>
<221>  CDS
<222>  (1)..(2367)

<400>  13
aag gag cac tac gag ttc aag aat gtc gcg atc ggc ggc ggc ggg tac       48
Lys Glu His Tyr Glu Phe Lys Asn Val Ala Ile Gly Gly Gly Gly Tyr
1               5                   10                  15

att acc ggg att gtc gcg cac cca aag acc aag gac ctg ctg tac gcg       96
Ile Thr Gly Ile Val Ala His Pro Lys Thr Lys Asp Leu Leu Tyr Ala
            20                  25                  30

cgc acg gac att ggc ggc gcg tac cgc tgg gac gca ggc acg tcc aag 144
Arg Thr Asp Ile Gly Gly Ala Tyr Arg Trp Asp Ala Gly Thr Ser Lys
        35                  40                  45

tgg atc ccg ctc aac gac ttt atc gag gcg cag gac atg aac att atg 192
Trp Ile Pro Leu Asn Asp Phe Ile Glu Ala Gln Asp Met Asn Ile Met
    50                  55                  60

ggc acc gag tcg atc gcg ctg gac ccc aac aac ccc gac agg ctg tac 240
Gly Thr Glu Ser Ile Ala Leu Asp Pro Asn Asn Pro Asp Arg Leu Tyr
65                  70                  75                  80

ctc gcg cag ggg cgc tat gtc ggc gac gag tgg gcg gcg ttc tat gtg 288
Leu Ala Gln Gly Arg Tyr Val Gly Asp Glu Trp Ala Ala Phe Tyr Val
                85                  90                  95

tcc gaa gac cgc ggc cag tcg ttt aca atc tac gag tcg ccg ttc ccg 336
Ser Glu Asp Arg Gly Gln Ser Phe Thr Ile Tyr Glu Ser Pro Phe Pro
            100                 105                 110

atg ggc gcc aac gac atg gga cgc aac aat ggc gag cgc ctc gct gtc 384
Met Gly Ala Asn Asp Met Gly Arg Asn Asn Gly Glu Arg Leu Ala Val
        115                 120                 125

aac ccg ttc aac tcg aac gag gtc tgg atg ggt acg cgt aca gag ggt 432
Asn Pro Phe Asn Ser Asn Glu Val Trp Met Gly Thr Arg Thr Glu Gly
    130                 135                 140

atc tgg aag agt tcg gac cgc gcc aag acc tgg aca aac gtc acg tcc 480
Ile Trp Lys Ser Ser Asp Arg Ala Lys Thr Trp Thr Asn Val Thr Ser
145                 150                 155                 160

atc ccg gac gcg ttc acc aac ggt atc gga tac acg tcg gtc att ttc 528
Ile Pro Asp Ala Phe Thr Asn Gly Ile Gly Tyr Thr Ser Val Ile Phe
                165                 170                 175

gac ccc gaa cgt aat ggc acc atc tac gcg agc gcg act gcc ccg cag 576
Asp Pro Glu Arg Asn Gly Thr Ile Tyr Ala Ser Ala Thr Ala Pro Gln
            180                 185                 190

ggc atg tac gtc acg cac gac ggc ggt gtc tcg tgg gag cca gtg gcg 624
Gly Met Tyr Val Thr His Asp Gly Gly Val Ser Trp Glu Pro Val Ala
        195                 200                 205

ggc cag ccg tcc agc tgg ctc aac agg acc acg ggc gcg ttc ccg gac 672
Gly Gln Pro Ser Ser Trp Leu Asn Arg Thr Thr Gly Ala Phe Pro Asp
    210                 215                 220

aag aag ccc gcg tcg atc gcg ccg cag ccc atg aaa gtc gct ctc acc 720
Lys Lys Pro Ala Ser Ile Ala Pro Gln Pro Met Lys Val Ala Leu Thr
225                 230                 235                 240

ccc aac ttc ctc tac gtg act tac gcc gac tac cct ggt cca tgg ggc 768
Pro Asn Phe Leu Tyr Val Thr Tyr Ala Asp Tyr Pro Gly Pro Trp Gly
                245                 250                 255

gtc acg ttc ggc gaa gtc tgg cgc cag aac cgc acc tcg ggc gcc tgg 816
Val Thr Phe Gly Glu Val Trp Arg Gln Asn Arg Thr Ser Gly Ala Trp
            260                 265                 270

gac gac att act ccc cgc gtc ggc aac tcg tcg cct gcc ccg tac aac 864
Asp Asp Ile Thr Pro Arg Val Gly Asn Ser Ser Pro Ala Pro Tyr Asn
        275                 280                 285

aac cag acg ttc cct gcg ggc gga ttt tgc ggt ctc agc gtc gac gcg 912
Asn Gln Thr Phe Pro Ala Gly Gly Phe Cys Gly Leu Ser Val Asp Ala
    290                 295                 300

acc aac ccc aac cgt ctc gtc gtc atc acc ctc gac cgc gac ccc gga 960
Thr Asn Pro Asn Arg Leu Val Val Ile Thr Leu Asp Arg Asp Pro Gly
305                 310                 315                 320

ccc gcc ctc gac agc atc tac ctc tca acc gat gcc ggc gcg acc tgg 1008
Pro Ala Leu Asp Ser Ile Tyr Leu Ser Thr Asp Ala Gly Ala Thr Trp
                325                 330                 335

aag gac gtc acc cag ctc tcg tcc ccg tcc aac ctc gaa ggt aac tgg 1056
Lys Asp Val Thr Gln Leu Ser Ser Pro Ser Asn Leu Glu Gly Asn Trp
            340                 345                 350

ggc cac ccg act aac gcg gcg cgg tac aag gac ggc acg cct gtt ccg 1104
Gly His Pro Thr Asn Ala Ala Arg Tyr Lys Asp Gly Thr Pro Val Pro
        355                 360                 365

tgg ctc gac ttc aac aac ggt ccc cag tgg ggg gga tac ggt gcg ccg 1152
Trp Leu Asp Phe Asn Asn Gly Pro Gln Trp Gly Gly Tyr Gly Ala Pro
    370                 375                 380

cac ggt acg ccc ggc ctc acc aag ttt ggc tgg tgg atg agc gct gtg 1200
His Gly Thr Pro Gly Leu Thr Lys Phe Gly Trp Trp Met Ser Ala Val
385                 390                 395                 400

ctt atc gat ccg ttc aac ccc gag cac ctg atg tac ggc acg ggg gcg 1248
Leu Ile Asp Pro Phe Asn Pro Glu His Leu Met Tyr Gly Thr Gly Ala
                405                 410                 415

acc atc tgg gcg acc gac acg ctc tcc cgt gtc gag aag gac tgg gcg 1296
Thr Ile Trp Ala Thr Asp Thr Leu Ser Arg Val Glu Lys Asp Trp Ala
            420                 425                 430

ccg agc tgg tac ctc cag atc gac ggt atc gag gag aat gcg atc ctg 1344
Pro Ser Trp Tyr Leu Gln Ile Asp Gly Ile Glu Glu Asn Ala Ile Leu
        435                 440                 445

tcg ctc cgc tcg ccc aag agc ggc gcg gcg ctc ctg tcg ggc atc ggt 1392
Ser Leu Arg Ser Pro Lys Ser Gly Ala Ala Leu Leu Ser Gly Ile Gly
    450                 455                 460

gac att agc ggc atg aag cac gac gac ctc acc aag ccc cag aag atg 1440
Asp Ile Ser Gly Met Lys His Asp Asp Leu Thr Lys Pro Gln Lys Met
465                 470                 475                 480

ttt ggt gcg ccc cag ttc tcc aac ctc gac agc atc gac gct gcg ggc 1488
Phe Gly Ala Pro Gln Phe Ser Asn Leu Asp Ser Ile Asp Ala Ala Gly
                485                 490                 495

aac ttc ccc aac gtt gtc gtc cgc gcc gga tcc tcg gga cac gag tac 1536
Asn Phe Pro Asn Val Val Val Arg Ala Gly Ser Ser Gly His Glu Tyr
            500                 505                 510

gac agc gcg tgc gcg cgc ggt gcg tac gcg act gac ggc gga gac gcg 1584

Asp Ser Ala Cys Ala Arg Gly Ala Tyr Ala Thr Asp Gly Gly Asp Ala
        515                 520                 525

tgg acc atc ttc cct acc tgc cct cct ggc atg aac gcg agc cac tac 1632
Trp Thr Ile Phe Pro Thr Cys Pro Pro Gly Met Asn Ala Ser His Tyr
    530                 535                 540

cag ggc agc acg att gca gtc gac gcg agc ggc agc cag atc gtg tgg 1680
Gln Gly Ser Thr Ile Ala Val Asp Ala Ser Gly Ser Gln Ile Val Trp
545                 550                 555                 560

tcg acc aag ctt gac gag cag gcc tcg gga ccg tgg tac tcg cac gac 1728
Ser Thr Lys Leu Asp Glu Gln Ala Ser Gly Pro Trp Tyr Ser His Asp
                565                 570                 575

tat ggc aag acg tgg tct gtt ccc gct ggc gac ctg aag gcc cag act 1776
Tyr Gly Lys Thr Trp Ser Val Pro Ala Gly Asp Leu Lys Ala Gln Thr
            580                 585                 590

gcc aat gtg ctc tcg gac aag gtc cag gat ggc acg ttc tac gct acc 1824
Ala Asn Val Leu Ser Asp Lys Val Gln Asp Gly Thr Phe Tyr Ala Thr
        595                 600                 605

gat ggc ggc aag ttc ttc gtc tcg acc gac ggc ggg aag tcg tat gcc 1872
Asp Gly Gly Lys Phe Phe Val Ser Thr Asp Gly Gly Lys Ser Tyr Ala
    610                 615                 620

gcc aag ggc gcc gga ctt gtc act ggc aca tcg ctc atg cct gcc gtg 1920
Ala Lys Gly Ala Gly Leu Val Thr Gly Thr Ser Leu Met Pro Ala Val
625                 630                 635                 640

aac ccc tgg gtg gcc ggc gac gtc tgg gtg cct gtt ccc gag ggc ggt 1968
Asn Pro Trp Val Ala Gly Asp Val Trp Val Pro Val Pro Glu Gly Gly
                645                 650                 655

ctc ttc cac tcg acc gac ttt ggc gcc tcg ttc acg agg gta ggt acc 2016
Leu Phe His Ser Thr Asp Phe Gly Ala Ser Phe Thr Arg Val Gly Thr
            660                 665                 670

gcc aac gcg acc ctc gtg agc gtc ggc gcc ccc aag tcc aag tcg gac 2064
Ala Asn Ala Thr Leu Val Ser Val Gly Ala Pro Lys Ser Lys Ser Asp
        675                 680                 685

ggc aag aag gct agc gcg ccc tcc gcg gtc ttc atc tgg ggc acc gac 2112
Gly Lys Lys Ala Ser Ala Pro Ser Ala Val Phe Ile Trp Gly Thr Asp
    690                 695                 700

aag cct gga agc gac atc ggc ctg tac cgc tcc gac gac aac ggc agc 2160
Lys Pro Gly Ser Asp Ile Gly Leu Tyr Arg Ser Asp Asp Asn Gly Ser
705                 710                 715                 720

acc tgg acg cgc gtc aat gac cag gag cac aac tac tcg ggc ccc acc 2208
Thr Trp Thr Arg Val Asn Asp Gln Glu His Asn Tyr Ser Gly Pro Thr
                725                 730                 735

atg atc gag gcc gac ccc aag gtc tac ggg cgc gtg tat cta ggc acg 2256
Met Ile Glu Ala Asp Pro Lys Val Tyr Gly Arg Val Tyr Leu Gly Thr
            740                 745                 750

aac ggc cgc ggt atc gtg tac gcc gac ctt acc aac aag aag agc aac 2304
Asn Gly Arg Gly Ile Val Tyr Ala Asp Leu Thr Asn Lys Lys Ser Asn

755                 760                 765

gag gag aag tcg acc gca aag tgc gcc aac ggc cag aag ggc acg cac     2352
Glu Glu Lys Ser Thr Ala Lys Cys Ala Asn Gly Gln Lys Gly Thr His
    770                 775                 780

tgc tat gtg aaa aag                                                 2367
Cys Tyr Val Lys Lys
785

<210>  14
<211>  789
<212>  PRT
<213>  Geotrichum sp. M128

<400>  14

Lys Glu His Tyr Glu Phe Lys Asn Val Ala Ile Gly Gly Gly Gly Tyr
1               5                   10                  15

Ile Thr Gly Ile Val Ala His Pro Lys Thr Lys Asp Leu Leu Tyr Ala
            20                  25                  30

Arg Thr Asp Ile Gly Gly Ala Tyr Arg Trp Asp Ala Gly Thr Ser Lys
        35                  40                  45

Trp Ile Pro Leu Asn Asp Phe Ile Glu Ala Gln Asp Met Asn Ile Met
    50                  55                  60

Gly Thr Glu Ser Ile Ala Leu Asp Pro Asn Asn Pro Asp Arg Leu Tyr
65                  70                  75                  80

Leu Ala Gln Gly Arg Tyr Val Gly Asp Glu Trp Ala Ala Phe Tyr Val
                85                  90                  95

Ser Glu Asp Arg Gly Gln Ser Phe Thr Ile Tyr Glu Ser Pro Phe Pro
            100                 105                 110

Met Gly Ala Asn Asp Met Gly Arg Asn Asn Gly Glu Arg Leu Ala Val
        115                 120                 125

Asn Pro Phe Asn Ser Asn Glu Val Trp Met Gly Thr Arg Thr Glu Gly
    130                 135                 140

Ile Trp Lys Ser Ser Asp Arg Ala Lys Thr Trp Thr Asn Val Thr Ser
145                 150                 155                 160

Ile Pro Asp Ala Phe Thr Asn Gly Ile Gly Tyr Thr Ser Val Ile Phe
                165                 170                 175

Asp Pro Glu Arg Asn Gly Thr Ile Tyr Ala Ser Ala Thr Ala Pro Gln
            180                 185                 190

Gly Met Tyr Val Thr His Asp Gly Gly Val Ser Trp Glu Pro Val Ala
        195                 200                 205

Gly Gln Pro Ser Ser Trp Leu Asn Arg Thr Thr Gly Ala Phe Pro Asp
    210                 215                 220

Lys Lys Pro Ala Ser Ile Ala Pro Gln Pro Met Lys Val Ala Leu Thr
225                 230                 235                 240

Pro Asn Phe Leu Tyr Val Thr Tyr Ala Asp Tyr Pro Gly Pro Trp Gly
                245                 250                 255

Val Thr Phe Gly Glu Val Trp Arg Gln Asn Arg Thr Ser Gly Ala Trp
            260                 265                 270

Asp Asp Ile Thr Pro Arg Val Gly Asn Ser Ser Pro Ala Pro Tyr Asn
        275                 280                 285

Asn Gln Thr Phe Pro Ala Gly Gly Phe Cys Gly Leu Ser Val Asp Ala
    290                 295                 300

Thr Asn Pro Asn Arg Leu Val Val Ile Thr Leu Asp Arg Asp Pro Gly
305                 310                 315                 320

Pro Ala Leu Asp Ser Ile Tyr Leu Ser Thr Asp Ala Gly Ala Thr Trp
                325                 330                 335

Lys Asp Val Thr Gln Leu Ser Ser Pro Ser Asn Leu Glu Gly Asn Trp
            340                 345                 350

Gly His Pro Thr Asn Ala Ala Arg Tyr Lys Asp Gly Thr Pro Val Pro
        355                 360                 365

Trp Leu Asp Phe Asn Asn Gly Pro Gln Trp Gly Gly Tyr Gly Ala Pro
    370                 375                 380

His Gly Thr Pro Gly Leu Thr Lys Phe Gly Trp Trp Met Ser Ala Val
385                 390                 395                 400

Leu Ile Asp Pro Phe Asn Pro Glu His Leu Met Tyr Gly Thr Gly Ala
                405                 410                 415

Thr Ile Trp Ala Thr Asp Thr Leu Ser Arg Val Glu Lys Asp Trp Ala
            420                 425                 430

Pro Ser Trp Tyr Leu Gln Ile Asp Gly Ile Glu Glu Asn Ala Ile Leu
        435                 440                 445

Ser Leu Arg Ser Pro Lys Ser Gly Ala Ala Leu Leu Ser Gly Ile Gly
    450                 455                 460

Asp Ile Ser Gly Met Lys His Asp Asp Leu Thr Lys Pro Gln Lys Met
465                 470                 475                 480

Phe Gly Ala Pro Gln Phe Ser Asn Leu Asp Ser Ile Asp Ala Ala Gly
                485                 490                 495

Asn Phe Pro Asn Val Val Val Arg Ala Gly Ser Ser Gly His Glu Tyr
            500                 505                 510

Asp Ser Ala Cys Ala Arg Gly Ala Tyr Ala Thr Asp Gly Gly Asp Ala
        515                 520                 525

Trp Thr Ile Phe Pro Thr Cys Pro Pro Gly Met Asn Ala Ser His Tyr
    530                 535                 540

Gln Gly Ser Thr Ile Ala Val Asp Ala Ser Gly Ser Gln Ile Val Trp
545                 550                 555                 560

Ser Thr Lys Leu Asp Glu Gln Ala Ser Gly Pro Trp Tyr Ser His Asp
                565                 570                 575

Tyr Gly Lys Thr Trp Ser Val Pro Ala Gly Asp Leu Lys Ala Gln Thr
            580                 585                 590

Ala Asn Val Leu Ser Asp Lys Val Gln Asp Gly Thr Phe Tyr Ala Thr
        595                 600                 605

Asp Gly Gly Lys Phe Phe Val Ser Thr Asp Gly Gly Lys Ser Tyr Ala
    610                 615                 620

Ala Lys Gly Ala Gly Leu Val Thr Gly Thr Ser Leu Met Pro Ala Val
625                 630                 635                 640

Asn Pro Trp Val Ala Gly Asp Val Trp Val Pro Val Pro Glu Gly Gly
                645                 650                 655

Leu Phe His Ser Thr Asp Phe Gly Ala Ser Phe Thr Arg Val Gly Thr

660                 665                 670

Ala Asn Ala Thr Leu Val Ser Val Gly Ala Pro Lys Ser Lys Ser Asp
        675                 680                 685

Gly Lys Lys Ala Ser Ala Pro Ser Ala Val Phe Ile Trp Gly Thr Asp
    690                 695                 700

Lys Pro Gly Ser Asp Ile Gly Leu Tyr Arg Ser Asp Asp Asn Gly Ser
705                 710                 715                 720

Thr Trp Thr Arg Val Asn Asp Gln Glu His Asn Tyr Ser Gly Pro Thr
                725                 730                 735

Met Ile Glu Ala Asp Pro Lys Val Tyr Gly Arg Val Tyr Leu Gly Thr
            740                 745                 750

Asn Gly Arg Gly Ile Val Tyr Ala Asp Leu Thr Asn Lys Lys Ser Asn
        755                 760                 765

Glu Glu Lys Ser Thr Ala Lys Cys Ala Asn Gly Gln Lys Gly Thr His
    770                 775                 780

Cys Tyr Val Lys Lys
785

<210>  15
<211>  40
<212>  DNA
<213>  Artificial Sequence

<220>
<223>  Primer

<400>  15
tcgctcgcgc atatgaagga gcactacgag ttcaagaatg                              40

<210>  16
<211>  38
<212>  DNA
<213>  Artificial Sequence

<220>
<223>  Primer

<400>  16
tggaagatct cctttttcac atagcagtgc gtgccctt                                38

<210>  17
<211>  2481

<212>  DNA
<213>  Artificial Sequence

<220>
<223>  Xyloglucan Oligosaccharide-Degrading Enzyme with Histidine Tag


<220>
<221>  CDS
<222>  (1)..(2481)

<400>  17
atg aag gag cac tac gag ttc aag aat gtc gcg atc ggc ggc ggc ggg      48
Met Lys Glu His Tyr Glu Phe Lys Asn Val Ala Ile Gly Gly Gly Gly
1               5                   10                  15

tac att acc ggg att gtc gcg cac cca aag acc aag gac ctg ctg tac      96
Tyr Ile Thr Gly Ile Val Ala His Pro Lys Thr Lys Asp Leu Leu Tyr
            20                  25                  30

gcg cgc acg gac att ggc ggc gcg tac cgc tgg gac gca ggc acg tcc      144
Ala Arg Thr Asp Ile Gly Gly Ala Tyr Arg Trp Asp Ala Gly Thr Ser
        35                  40                  45

aag tgg atc ccg ctc aac gac ttt atc gag gcg cag gac atg aac att      192
Lys Trp Ile Pro Leu Asn Asp Phe Ile Glu Ala Gln Asp Met Asn Ile
    50                  55                  60

atg ggc acc gag tcg atc gcg ctg gac ccc aac aac ccc gac agg ctg      240
Met Gly Thr Glu Ser Ile Ala Leu Asp Pro Asn Asn Pro Asp Arg Leu
65                  70                  75                  80

tac ctc gcg cag ggg cgc tat gtc ggc gac gag tgg gcg gcg ttc tat      288
Tyr Leu Ala Gln Gly Arg Tyr Val Gly Asp Glu Trp Ala Ala Phe Tyr
                85                  90                  95

gtg tcc gaa gac cgc ggc cag tcg ttt aca atc tac gag tcg ccg ttc      336
Val Ser Glu Asp Arg Gly Gln Ser Phe Thr Ile Tyr Glu Ser Pro Phe
            100                 105                 110

ccg atg ggc gcc aac gac atg gga cgc aac aat ggc gag cgc ctc gct      384
Pro Met Gly Ala Asn Asp Met Gly Arg Asn Asn Gly Glu Arg Leu Ala
        115                 120                 125

gtc aac ccg ttc aac tcg aac gag gtc tgg atg ggt acg cgt aca gag      432
Val Asn Pro Phe Asn Ser Asn Glu Val Trp Met Gly Thr Arg Thr Glu
    130                 135                 140

ggt atc tgg aag agt tcg gac cgc gcc aag acc tgg aca aac gtc acg      480
Gly Ile Trp Lys Ser Ser Asp Arg Ala Lys Thr Trp Thr Asn Val Thr
145                 150                 155                 160

tcc atc ccg gac gcg ttc acc aac ggt atc gga tac acg tcg gtc att      528
Ser Ile Pro Asp Ala Phe Thr Asn Gly Ile Gly Tyr Thr Ser Val Ile
                165                 170                 175

ttc gac ccc gaa cgt aat ggc acc atc tac gcg agc gcg act gcc ccg      576
Phe Asp Pro Glu Arg Asn Gly Thr Ile Tyr Ala Ser Ala Thr Ala Pro
            180                 185                 190

cag ggc atg tac gtc acg cac gac ggc ggt gtc tcg tgg gag cca gtg      624

Gln Gly Met Tyr Val Thr His Asp Gly Gly Val Ser Trp Glu Pro Val
        195                 200                 205

gcg ggc cag ccg tcc agc tgg ctc aac agg acc acg ggc gcg ttc ccg 672
Ala Gly Gln Pro Ser Ser Trp Leu Asn Arg Thr Thr Gly Ala Phe Pro
    210                 215                 220

gac aag aag ccc gcg tcg atc gcg ccg cag ccc atg aaa gtc gct ctc 720
Asp Lys Lys Pro Ala Ser Ile Ala Pro Gln Pro Met Lys Val Ala Leu
225                 230                 235                 240

acc ccc aac ttc ctc tac gtg act tac gcc gac tac cct ggt cca tgg 768
Thr Pro Asn Phe Leu Tyr Val Thr Tyr Ala Asp Tyr Pro Gly Pro Trp
                245                 250                 255

ggc gtc acg ttc ggc aaa gtc tgg cgc cag aac cgc acc tcg ggc gcc 816
Gly Val Thr Phe Gly Lys Val Trp Arg Gln Asn Arg Thr Ser Gly Ala
            260                 265                 270

tgg gac gac att act ccc cgc gtc ggc aac tcg tcg cct gcc ccg tac 864
Trp Asp Asp Ile Thr Pro Arg Val Gly Asn Ser Ser Pro Ala Pro Tyr
        275                 280                 285

aac aac cag acg ttc cct gcg ggc gga ttt tgc ggt ctc agc gtc gac 912
Asn Asn Gln Thr Phe Pro Ala Gly Gly Phe Cys Gly Leu Ser Val Asp
    290                 295                 300

gcg acc aac ccc aac cgt ctc gtc gtc atc acc ctc gac cgc gac ccc 960
Ala Thr Asn Pro Asn Arg Leu Val Val Ile Thr Leu Asp Arg Asp Pro
305                 310                 315                 320

gga ccc gcc ctc gac agc atc tac ctc tca acc gat gcc ggc gcg acc 1008
Gly Pro Ala Leu Asp Ser Ile Tyr Leu Ser Thr Asp Ala Gly Ala Thr
                325                 330                 335

tgg aag gac gtc acc cag ctc tcg tcc ccg tcc aac ctc gaa ggt aac 1056
Trp Lys Asp Val Thr Gln Leu Ser Ser Pro Ser Asn Leu Glu Gly Asn
            340                 345                 350

tgg ggc cac ccg act aac gcg gcg cgg tac aag gac ggc acg cct gtt 1104
Trp Gly His Pro Thr Asn Ala Ala Arg Tyr Lys Asp Gly Thr Pro Val
        355                 360                 365

ccg tgg ctc gac ttc aac aac ggt ccc cag tgg ggg gga tac ggt gcg 1152
Pro Trp Leu Asp Phe Asn Asn Gly Pro Gln Trp Gly Gly Tyr Gly Ala
    370                 375                 380

ccg cac ggt acg ccc ggc ctc acc aag ttt ggc tgg tgg atg agc gct 1200
Pro His Gly Thr Pro Gly Leu Thr Lys Phe Gly Trp Trp Met Ser Ala
385                 390                 395                 400

gtg ctt atc gat ccg ttc aac ccc gag cac ctg atg tac ggc acg ggg 1248
Val Leu Ile Asp Pro Phe Asn Pro Glu His Leu Met Tyr Gly Thr Gly
                405                 410                 415

gcg acc atc tgg gcg acc gac acg ctc tcc cgt gtc gag aag gac tgg 1296
Ala Thr Ile Trp Ala Thr Asp Thr Leu Ser Arg Val Glu Lys Asp Trp
            420                 425                 430

gcg ccg agc tgg tac ctc cag atc gac ggt atc gag gag aat gcg atc 1344
Ala Pro Ser Trp Tyr Leu Gln Ile Asp Gly Ile Glu Glu Asn Ala Ile

        435                 440                 445

ctg tcg ctc cgc tcg ccc aag agc ggc gcg gcg ctc ctg tcg ggc atc 1392
Leu Ser Leu Arg Ser Pro Lys Ser Gly Ala Ala Leu Leu Ser Gly Ile
    450                 455                 460

ggt gac att agc ggc atg aag cac gac gac ctc acc aag ccc cag aag 1440
Gly Asp Ile Ser Gly Met Lys His Asp Asp Leu Thr Lys Pro Gln Lys
465                 470                 475                 480

atg ttt ggt gcg ccc cag ttc tcc aac ctc gac agc atc gac gct gcg 1488
Met Phe Gly Ala Pro Gln Phe Ser Asn Leu Asp Ser Ile Asp Ala Ala
                485                 490                 495

ggc aac ttc ccc aac gtt gtc gtc cgc gcc gga tcc tcg gga cac gag 1536
Gly Asn Phe Pro Asn Val Val Val Arg Ala Gly Ser Ser Gly His Glu
            500                 505                 510

tac gac agc gcg tgc gcg cgc ggt gcg tac gcg act gac ggc gga gac 1584
Tyr Asp Ser Ala Cys Ala Arg Gly Ala Tyr Ala Thr Asp Gly Gly Asp
        515                 520                 525

gcg tgg acc atc ttc cct acc tgc cct cct ggc atg aac gcg agc cac 1632
Ala Trp Thr Ile Phe Pro Thr Cys Pro Pro Gly Met Asn Ala Ser His
    530                 535                 540

tac cag ggc agc acg att gca gtc gac gcg agc ggc agc cag atc gtg 1680
Tyr Gln Gly Ser Thr Ile Ala Val Asp Ala Ser Gly Ser Gln Ile Val
545                 550                 555                 560

tgg tcg acc aag ctt gac gag cag gcc tcg gga ccg tgg tac tcg cac 1728
Trp Ser Thr Lys Leu Asp Glu Gln Ala Ser Gly Pro Trp Tyr Ser His
                565                 570                 575

gac tat ggc aag acg tgg tct gtt ccc gct ggc gac ctg aag gcc cag 1776
Asp Tyr Gly Lys Thr Trp Ser Val Pro Ala Gly Asp Leu Lys Ala Gln
            580                 585                 590

act gcc aat gtg ctc tcg gac aag gtc cag gat ggc acg ttc tac gct 1824
Thr Ala Asn Val Leu Ser Asp Lys Val Gln Asp Gly Thr Phe Tyr Ala
        595                 600                 605

acc gat ggc ggc aag ttc ttc gtc tcg acc gac ggc ggg aag tcg tat 1872
Thr Asp Gly Gly Lys Phe Phe Val Ser Thr Asp Gly Gly Lys Ser Tyr
    610                 615                 620

gcc gcc aag ggc gcc gga ctt gtc act ggc aca tcg ctc atg cct gcc 1920
Ala Ala Lys Gly Ala Gly Leu Val Thr Gly Thr Ser Leu Met Pro Ala
625                 630                 635                 640

gtg aac ccc tgg gtg gcc ggc gac gtc tgg gtg cct gtt ccc gag ggc 1968
Val Asn Pro Trp Val Ala Gly Asp Val Trp Val Pro Val Pro Glu Gly
                645                 650                 655

ggt ctc ttc cac tcg acc gac ttt ggc gcc tcg ttc acg agg gta ggt 2016
Gly Leu Phe His Ser Thr Asp Phe Gly Ala Ser Phe Thr Arg Val Gly
            660                 665                 670

acc gcc aac gcg acc ctc gtg agc gtc ggc gcc ccc aag tcc aag tcg 2064
Thr Ala Asn Ala Thr Leu Val Ser Val Gly Ala Pro Lys Ser Lys Ser
        675                 680                 685

gac ggc aag aag gct agc gcg ccc tcc gcg gtc ttc atc tgg ggc acc     2112
Asp Gly Lys Lys Ala Ser Ala Pro Ser Ala Val Phe Ile Trp Gly Thr
    690                 695                 700

gac aag cct gga agc gac atc ggc ctg tac cgc tcc gac gac aac ggc     2160
Asp Lys Pro Gly Ser Asp Ile Gly Leu Tyr Arg Ser Asp Asp Asn Gly
705                 710                 715                 720

agc acc tgg acg cgc gtc aat gac cag gag cac aac tac tcg ggc ccc     2208
Ser Thr Trp Thr Arg Val Asn Asp Gln Glu His Asn Tyr Ser Gly Pro
                725                 730                 735

acc atg atc gag gcc gac ccc aag gtc tac ggg cgc gtg tat cta ggc     2256
Thr Met Ile Glu Ala Asp Pro Lys Val Tyr Gly Arg Val Tyr Leu Gly
            740                 745                 750

acg aac ggc cgc ggt atc gtg tac gcc gac ctt acc aac aag aag agc     2304
Thr Asn Gly Arg Gly Ile Val Tyr Ala Asp Leu Thr Asn Lys Lys Ser
        755                 760                 765

aac gag gag aag tcg acc gca aag tgc gcc aac ggc cag aag ggc acg     2352
Asn Glu Glu Lys Ser Thr Ala Lys Cys Ala Asn Gly Gln Lys Gly Thr
    770                 775                 780

cac tgc tat gtg aaa aag gag atc tgg gta ccc tgg tgc cac gcg gtt     2400
His Cys Tyr Val Lys Lys Glu Ile Trp Val Pro Trp Cys His Ala Val
785                 790                 795                 800

cca tgg ctg ata tcg gat ccg aat tcg agc tcc gtc gac aag ctt gcg     2448
Pro Trp Leu Ile Ser Asp Pro Asn Ser Ser Ser Val Asp Lys Leu Ala
                805                 810                 815

gcc gca ctc gag cac cac cac cac cac cac tga                         2481
Ala Ala Leu Glu His His His His His His
            820                 825

<210>  18
<211>  826
<212>  PRT
<213>  Artificial Sequence

<220>
<223>  Xyloglucan Oligosaccharide-Degrading Enzyme with Histidine Tag

<400>  18

Met Lys Glu His Tyr Glu Phe Lys Asn Val Ala Ile Gly Gly Gly Gly
1               5                   10                  15

Tyr Ile Thr Gly Ile Val Ala His Pro Lys Thr Lys Asp Leu Leu Tyr
            20                  25                  30

Ala Arg Thr Asp Ile Gly Gly Ala Tyr Arg Trp Asp Ala Gly Thr Ser
        35                  40                  45

Lys Trp Ile Pro Leu Asn Asp Phe Ile Glu Ala Gln Asp Met Asn Ile

50                  55                  60

Met Gly Thr Glu Ser Ile Ala Leu Asp Pro Asn Asn Pro Asp Arg Leu
65                  70                  75                  80

Tyr Leu Ala Gln Gly Arg Tyr Val Gly Asp Glu Trp Ala Ala Phe Tyr
                85                  90                  95

Val Ser Glu Asp Arg Gly Gln Ser Phe Thr Ile Tyr Glu Ser Pro Phe
            100                 105                 110

Pro Met Gly Ala Asn Asp Met Gly Arg Asn Asn Gly Glu Arg Leu Ala
        115                 120                 125

Val Asn Pro Phe Asn Ser Asn Glu Val Trp Met Gly Thr Arg Thr Glu
    130                 135                 140

Gly Ile Trp Lys Ser Ser Asp Arg Ala Lys Thr Trp Thr Asn Val Thr
145                 150                 155                 160

Ser Ile Pro Asp Ala Phe Thr Asn Gly Ile Gly Tyr Thr Ser Val Ile
                165                 170                 175

Phe Asp Pro Glu Arg Asn Gly Thr Ile Tyr Ala Ser Ala Thr Ala Pro
            180                 185                 190

Gln Gly Met Tyr Val Thr His Asp Gly Gly Val Ser Trp Glu Pro Val
        195                 200                 205

Ala Gly Gln Pro Ser Ser Trp Leu Asn Arg Thr Thr Gly Ala Phe Pro
    210                 215                 220

Asp Lys Lys Pro Ala Ser Ile Ala Pro Gln Pro Met Lys Val Ala Leu
225                 230                 235                 240

Thr Pro Asn Phe Leu Tyr Val Thr Tyr Ala Asp Tyr Pro Gly Pro Trp
                245                 250                 255

Gly Val Thr Phe Gly Lys Val Trp Arg Gln Asn Arg Thr Ser Gly Ala
            260                 265                 270

Trp Asp Asp Ile Thr Pro Arg Val Gly Asn Ser Ser Pro Ala Pro Tyr
        275                 280                 285

Asn Asn Gln Thr Phe Pro Ala Gly Gly Phe Cys Gly Leu Ser Val Asp
    290                 295                 300

Ala Thr Asn Pro Asn Arg Leu Val Val Ile Thr Leu Asp Arg Asp Pro
305                 310                 315                 320

Gly Pro Ala Leu Asp Ser Ile Tyr Leu Ser Thr Asp Ala Gly Ala Thr
                325                 330                 335

Trp Lys Asp Val Thr Gln Leu Ser Ser Pro Ser Asn Leu Glu Gly Asn
            340                 345                 350

Trp Gly His Pro Thr Asn Ala Ala Arg Tyr Lys Asp Gly Thr Pro Val
        355                 360                 365

Pro Trp Leu Asp Phe Asn Asn Gly Pro Gln Trp Gly Gly Tyr Gly Ala
    370                 375                 380

Pro His Gly Thr Pro Gly Leu Thr Lys Phe Gly Trp Trp Met Ser Ala
385                 390                 395                 400

Val Leu Ile Asp Pro Phe Asn Pro Glu His Leu Met Tyr Gly Thr Gly
                405                 410                 415

Ala Thr Ile Trp Ala Thr Asp Thr Leu Ser Arg Val Glu Lys Asp Trp
            420                 425                 430

Ala Pro Ser Trp Tyr Leu Gln Ile Asp Gly Ile Glu Glu Asn Ala Ile
        435                 440                 445

Leu Ser Leu Arg Ser Pro Lys Ser Gly Ala Ala Leu Leu Ser Gly Ile
    450                 455                 460

Gly Asp Ile Ser Gly Met Lys His Asp Asp Leu Thr Lys Pro Gln Lys
465                 470                 475                 480

Met Phe Gly Ala Pro Gln Phe Ser Asn Leu Asp Ser Ile Asp Ala Ala
                485                 490                 495

Gly Asn Phe Pro Asn Val Val Val Arg Ala Gly Ser Ser Gly His Glu
            500                 505                 510

Tyr Asp Ser Ala Cys Ala Arg Gly Ala Tyr Ala Thr Asp Gly Gly Asp
        515                 520                 525

Ala Trp Thr Ile Phe Pro Thr Cys Pro Pro Gly Met Asn Ala Ser His
    530                 535                 540

Tyr Gln Gly Ser Thr Ile Ala Val Asp Ala Ser Gly Ser Gln Ile Val
545                 550                 555                 560

Trp Ser Thr Lys Leu Asp Glu Gln Ala Ser Gly Pro Trp Tyr Ser His
                565                 570                 575

Asp Tyr Gly Lys Thr Trp Ser Val Pro Ala Gly Asp Leu Lys Ala Gln
            580                 585                 590

Thr Ala Asn Val Leu Ser Asp Lys Val Gln Asp Gly Thr Phe Tyr Ala
        595                 600                 605

Thr Asp Gly Gly Lys Phe Phe Val Ser Thr Asp Gly Gly Lys Ser Tyr
    610                 615                 620

Ala Ala Lys Gly Ala Gly Leu Val Thr Gly Thr Ser Leu Met Pro Ala
625                 630                 635                 640

Val Asn Pro Trp Val Ala Gly Asp Val Trp Val Pro Val Pro Glu Gly
                645                 650                 655

Gly Leu Phe His Ser Thr Asp Phe Gly Ala Ser Phe Thr Arg Val Gly
            660                 665                 670

Thr Ala Asn Ala Thr Leu Val Ser Val Gly Ala Pro Lys Ser Lys Ser
        675                 680                 685

Asp Gly Lys Lys Ala Ser Ala Pro Ser Ala Val Phe Ile Trp Gly Thr
    690                 695                 700

Asp Lys Pro Gly Ser Asp Ile Gly Leu Tyr Arg Ser Asp Asp Asn Gly
705                 710                 715                 720

Ser Thr Trp Thr Arg Val Asn Asp Gln Glu His Asn Tyr Ser Gly Pro
                725                 730                 735

Thr Met Ile Glu Ala Asp Pro Lys Val Tyr Gly Arg Val Tyr Leu Gly
            740                 745                 750

Thr Asn Gly Arg Gly Ile Val Tyr Ala Asp Leu Thr Asn Lys Lys Ser
        755                 760                 765

Asn Glu Glu Lys Ser Thr Ala Lys Cys Ala Asn Gly Gln Lys Gly Thr
    770                 775                 780

His Cys Tyr Val Lys Lys Glu Ile Trp Val Pro Trp Cys His Ala Val
785                 790                 795                 800

Pro Trp Leu Ile Ser Asp Pro Asn Ser Ser Ser Val Asp Lys Leu Ala
                805                 810                 815

Ala Ala Leu Glu His His His His His His
            820                 825

<210>  19
<211>  18
<212>  DNA
<213>  Artificial Sequence

<220>
<223>  Histidine tag

<400>  19
caycaycayc aycaycay                                                    18

## Claims

1. A purified enzyme which specifically cleaves the second β-glucoside linkage counted from the reducing end among the β-glucoside linkages constituting the principal chain of xyloglucan oligosaccharide.

2. A purified polypeptide comprising the amino acid sequence shown in SEQ ID NO: 14, or an amino acid sequence having one or more amino acid residue deletions, additions, insertions or substitutions relative to the amino acid sequence shown in SEQ ID NO: 14, said polypeptide having a xyloglucan oligosaccharide-degradation activity.

3. The purified polypeptide as defined in claim 2, which further includes methionine at the N-terminus thereof.

4. The purified polypeptide as defined in claim 2, which further includes a signal sequence.

5. A purified polypeptide comprising the amino acid sequence shown in SEQ ID NO: 12, or an amino acid sequence having one or more amino acid deletions, additions, insertions or substitutions relative to the amino acid sequence shown in SEQ ID NO: 12, said polypeptide serving as a precursor of a polypeptide having a xyloglucan oligosaccharide-degradation activity.

6. The purified polypeptide as defined in claim 3, wherein said amino acid sequence is the amino acid sequence shown in SEQ ID NO: 18.

7. An isolated polynucleotide encoding the polypeptide as defined in any one of claims 2 to 5.

8. An isolated polynucleotide comprising the base sequence shown in SEQ ID NO: 13, or a base sequence having one or more nucleic acid deletions, additions, insertions or substitutions relative to the base sequence shown in SEQ ID NO: 13, said polynucleotide encoding a polypeptide having a xyloglucan oligosaccharide-degradation activity.

9. The isolated polynucleotide as defined in claim 8, which further includes an initiation codon.

10. The isolated polynucleotide as defined in claim 8, which further include a base sequence corresponding to a signal peptide sequence.

11. An isolated polynucleotide comprising the base sequence shown in SEQ ID NO: 11, or a base sequence having one or more nucleic acid deletions, additions, insertions or substitutions relative to the base sequence shown in SEQ ID NO: 11, said polynucleotide encoding a precursor of a polypeptide having a xyloglucan oligosaccharide-degradation activity.

12. An isolated polynucleotide which hybridizes to a polynucleotide as defined in any one of claims 8 to 11, under stringent conditions.

13. An isolated polynucleotide having homology to a polynucleotide as defined in any one of claims 8 to 11.

14. An isolated polynucleotide which is a degenerate of a polynucleotide as defined in any one of claims 8 to 11.

15. A recombinant vector comprising a polynucleotide as defined in any one of claims 8 to 11.

16. A transformant comprising the recombinant vector as defined in claim 15.

17. A method of preparing a polypeptide, said method comprising culturing the transformant as defined in claim 16 under conditions such that said transformant produces the polypeptide encoded by the polynucleotide, and collecting the polypeptide so expressed.

18. The method as defined in claim 17, wherein said polypeptide has a xyloglucan oligosaccharide-degradation activity.

19. The method as defined in claim 18, said xyloglucan oligosaccharide-degradation activity is operative to specifically cleave the second β-glucoside linkage counted from the reducing end among the β-glucoside linkages constituting the principal chain of xyloglucan oligosaccharide.

20. A purified polypeptide consisting of amino acid residues 1 to 789 of SEQ ID NO: 14.

21. A purified polypeptide consisting of amino acid residues 1 to 812 of SEQ ID NO: 12.

22. An isolated polynucleotide consisting of nucleic acid residues 1 to 2367 of SEQ ID NO: 13.

23. An isolated polynucleotide consisting of nucleic acid residues 1 to 2646 of SEQ ID NO: 11.
